# EUROPEAN PATENT APPLICATION

(11) **EP 4 159 031 A1**
(43) Date of publication of application: **05.04.2023**
(21) Application number: 21841528.9
(22) Date of filing: 13.07.2021
(51) Int. Cl.: A01K 41/00, G06Q 50/10

(54) **EGG MANAGEMENT SUPPORT SYSTEM**

(30) Priority: 17.07.2020 JP 2020122962
(71) Applicant: Nabel Co., Ltd., Kyoto-shi, Kyoto 601-8444 (JP)
(72) Inventor: NAMBU, Takahiko, Kyoto-shi, Kyoto 601-8444 (JP)
(74) Representative: Prüfer & Partner mbB Patentanwälte · Rechtsanwälte
(86) International application number: PCT/JP2021/026261
(87) International publication number: WO 2022/014578

(57) **Abstract**

An egg management support system (1) in the present invention includes a receiver (11) and a storage (12). The receiver (11) receives from an information processing apparatus (2) at a hatchery (4), biological information of an egg measured before hatching and incubation performance obtained for a chick that has hatched out of the egg. In the storage (12), the biological information and the incubation performance are stored in association. Thus, the egg management support system capable of effectively making use of information obtained from the hatchery can be provided.

## Description

### TECHNICAL FIELD

The present invention relates to an egg management support system.

### BACKGROUND ART

An exemplary egg management support system that uses information on a temperature around an eggshell obtained from a temperature sensor attached to a tray placed in an incubator has been known (see, for example, PTL 1). This egg management support system controls an environment in the incubator such that a temperature in the incubator is brought closer to a target temperature in the incubator by comparing a current temperature in the incubator with the target temperature in the incubator. In the conventional egg management support system, feedback has been given to the incubator the same as the incubator from which temperature information is collected.

### CITATION LIST

### PATENT LITERATURE

PTL 1: US Patent No. 7,950,350

### SUMMARY OF INVENTION

### TECHNICAL PROBLEM

With development and sophistication of information technology in recent years, in various fields, a tendency to analyze, as what is called big data, information obtained from inspection apparatuses and the like and to make use of the big data for development of new products and services has been noticeable. In the field relating to incubation as well, there are needs for management of eggs by making use of information obtained from a hatchery.

Nothing has conventionally satisfied such needs, and each hatchery has managed eggs, relying on experiences and intuition. The present invention aims to effectively make use of information obtained from a hatchery.

### SOLUTION TO PROBLEM

An egg management support system in the present invention includes a receiver and a storage. The receiver receives from an information processing apparatus at a hatchery, biological information of an egg measured before hatching and incubation performance obtained for a chick that has hatched out of the egg. In the storage, the biological information and the incubation performance are stored in association.

Even though eggs belong to the same lot, the "biological information of an egg" is different for each egg and it can be measured with a measurement unit. For example, exemplary biological information includes an egg weight, an eggshell temperature, an egg shape index (calculated by dividing a short radius of an egg by a long radius and multiplying the result by 100), an air cell height, an air cell volume, an eggshell thickness, strength of an eggshell, fetal movement of an embryo, and a heartbeat.

The "incubation performance" means information on a condition after hatching and means a state of a chick identifiable during a period from hatching until shipping. Exemplary incubation performance includes a hatching rate, a hatch window, and chick quality. The incubation performance may be information for each individual chick or information per lot. The hatch window generally refers to an indicator that represents a time span between hatching of a first chick and hatching of a last chick, which originates from a difference in timing of hatching among a plurality of eggs placed in a specific hatcher tray.

### ADVANTAGEOUS EFFECTS OF INVENTION

According to the present invention, the egg management support system capable of effectively making use of information obtained from a hatchery can be provided.

The foregoing and other objects, features, aspects and advantages of this invention will become apparent from the following detailed description of this invention when taken in conjunction with the accompanying drawings.

### BRIEF DESCRIPTION OF DRAWINGS

Fig. 1 is a diagram showing a chick production process in a general hatchery.
Fig. 2 is a diagram showing overview of an egg management support system according to one embodiment of the present invention.
Fig. 3 is a diagram showing overview of the egg management support system according to the embodiment.

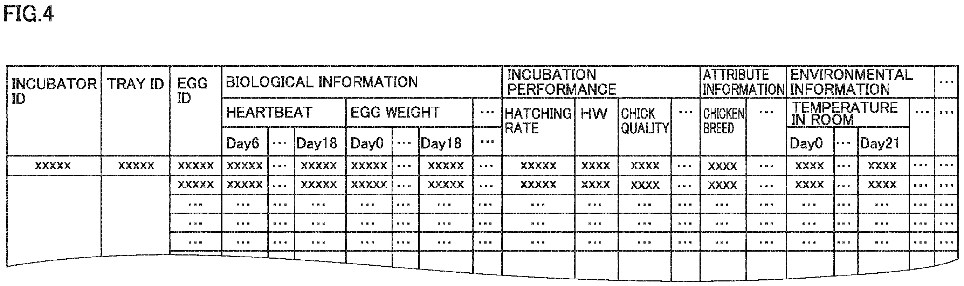

Fig. 4 shows a table of contents stored in a storage according to the embodiment.
Fig. 5 is a diagram showing overview of an egg management support system according to a modification of the present invention.
Fig. 6 shows a table of contents stored in the storage according to the modification of the present invention.

### DESCRIPTION OF EMBODIMENTS

An embodiment of the present invention will be described below with reference to the drawings.

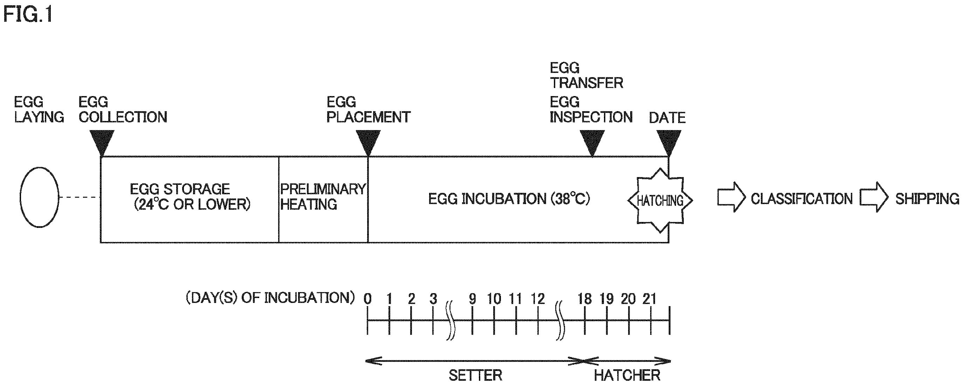

In a breeder company, a farm where a group of breeding hens which are parents of layer chickens is bred is called a breeder farm. As shown in Fig. 1, eggs laid in this breeder farm are once gathered into an egg storage location in a hatchery 4, thereafter go through a process called preparatory heating, and are placed in an incubator 3 (egg placement), and incubation is started. Inspection is conducted on an eighteenth or nineteenth day after egg placement in incubator 3, and the eggs are hatched on an approximately twenty-first day after egg placement. After hatching, chicks are classified in a chick treatment apparatus 5 and shipped to a farm. In chick treatment apparatus 5, the number of chicks is counted. In chick treatment apparatus 5, chicks are classified into male chicks and female chicks. Even when chicks are female, those with a crooked leg, physical weakness, or a malformation are screened out. Classification is done by using an image of chicks or manually.

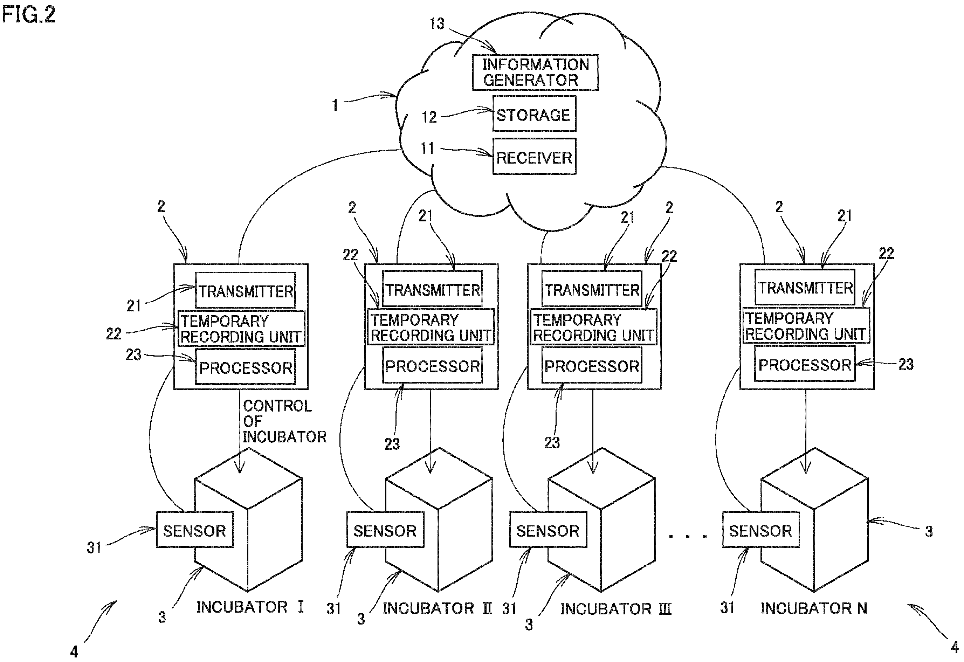

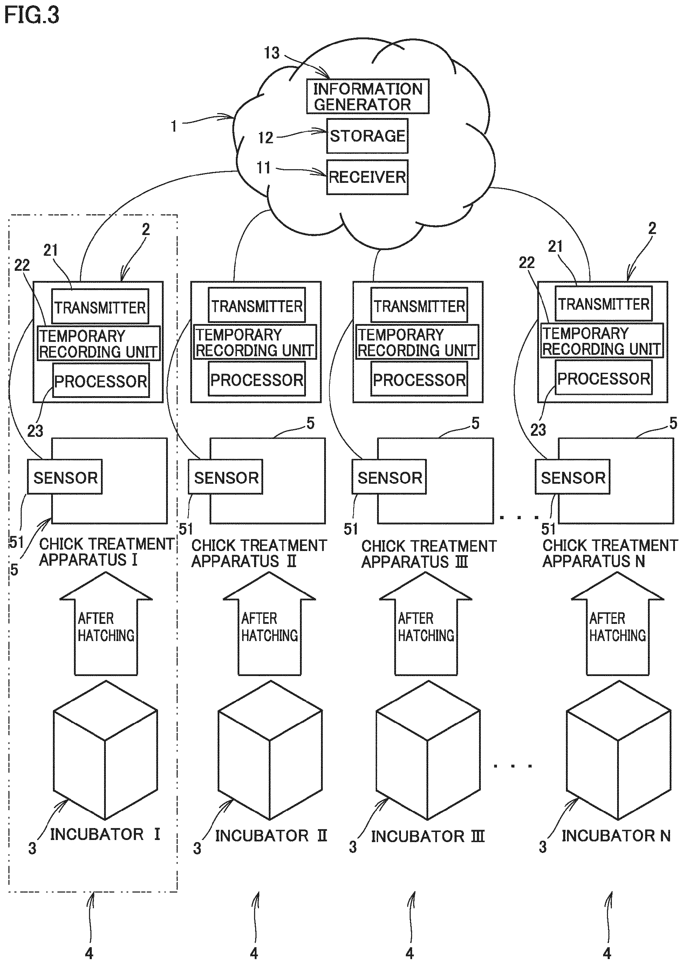

The present embodiment is directed to application of an egg management support system 1 to be used for a plurality of hatcheries 4 to an environment at hatchery 4 as shown in Figs. 2 and 3. Egg management support system 1 uses a computer system to store information for each hatchery and calculate information that can be used for production of better chicks. This egg management support system 1 uses cloud computing to support management of production of mid-hatch eggs placed in incubator 3. The "mid-hatch eggs" refer to eggs in the course from placement in incubator 3 until hatching, and an Xth day after egg placement in incubator 3 is denoted as an "Xth day of incubation" or "Day X."

As shown in Figs. 2 and 3, information obtained at hatchery 4 is stored in egg management support system 1 via an information processing apparatus 2. Incubator 3 provided with a sensor 31, information processing apparatus 2, and egg management support system 1 are connected to communicate with one another over a network such as the Internet.

Egg management support system 1 includes a receiver 11, a storage 12, and an information generator 13. Receiver 11, storage 12, and information generator 13 may be configured with a physically integrated computer or with physically separate computers. Egg management support system 1 is connected to communicate with information processing apparatuses 2, incubators 3, or chick treatment apparatuses 5 over the network. As shown in Figs. 2 and 3, a plurality of incubators I, II, III, ..., and N are placed in respective different hatcheries 4.

Receiver 11 receives from information processing apparatuses 2 at a plurality of hatcheries 4, biological information of eggs measured on each of a plurality of days until hatching and incubation performance obtained for chicks that have hatched out of the eggs. Specifically, receiver 11 receives biological information, incubation performance, attribute information, and environmental information over the network from information processing apparatuses 2. Exemplary biological information includes an egg weight, an eggshell temperature, an egg shape index, an air cell height, an air cell volume, an eggshell thickness, strength of an eggshell, fetal movement of an embryo, and a heartbeat. The incubation performance includes a hatching rate (whether or not a chick has hatched or a rate of hatching in a group of eggs), a hatch window (whether a chick has hatched earlier or later, or narrowness of a hatch window of a group of eggs; denoted as "HW" in the drawings), chick quality (whether or not a chick is good in quality), and a sex (whether a chick is male or female, or a ratio of one sex in a group of eggs). The attribute information is common among eggs in the same lot and includes genetic information and growth information. The genetic information includes a chicken breed. The growth information includes age in day of a parent chicken, time and day of egg laying, contents of a feed, and a disease history and a vaccination history of a parent chicken. The environmental information includes a temperature and a humidity in incubator 3. The biological information and the environmental information are information obtained from sensor 31 and time and day information is associated with such information.

Storage 12 is a cloud storage accessible over the network by authenticated information processing apparatus 2 including a producer of chicks. The biological information and the incubation performance are stored in storage 12 in association. The attribute information and the environmental information are also stored in storage 12 in association with them. Information generated by information generator 13 is also stored in storage 12.

Information generator 13 generates environment control information for controlling an inside environment in incubator 3 based on the biological information and the incubation performance.

Incubator 3 placed at hatchery 4 includes various sensors 31. Sensor 31 measures, for example, a state of an egg (an eggshell temperature and a heartbeat) and the biological information is obtained from sensor 31. Another sensor 31 measures, for example, an environment in incubator 3 (a temperature and a humidity in incubator 3) and the environmental information is obtained from this sensor 31. Timing of hatching and incubation performance may be obtained from sensor 31 that measures a humidity in incubator 3.

Chick treatment apparatus 5 placed at hatchery 4 includes various sensors 51. Sensor 51 measures, for example, a state of a chick and the incubation performance is obtained from this sensor 51. The incubation performance may be an input of a result of observation by a worker.

The attribute information is inputted by a worker. The attribute information may be obtained by reading with a reader, of a bar code provided in a tray where eggs are accommodated.

Information processing apparatus 2 includes a transmitter 21, a temporary recording unit 22, and a processor 23. Transmitter 21 transmits to egg management support system 1, information received from sensors 31 and 51, a result of calculation by processor 23, and information inputted by a worker. Information to be transmitted to transmitter 21 is temporarily recorded in temporary recording unit 22. Processor 23 performs arithmetic processing of data in temporary recording unit 22 with the use of a prescribed program. Processor 23 controls incubator 3 based on the environment control information generated by information generator 13.

A method of using egg management support system 1 will now be described.

Initially, at a time point of Day 0 when eggs are placed in incubator 3, a bar code provided in a tray is read with a reader. A tray ID and a chicken breed are linked to the bar code. Information on the tray ID and the chicken breed read with the reader is recorded in temporary recording unit 22 in information processing apparatus 2 and transmitted over the network to egg management support system 1.

A heartbeat and a weight of each of a plurality of eggs placed in one tray are measured. An egg ID is provided to each measured egg. The heartbeat is measured by emitting light to an egg and detecting light diffused in the inside of the egg. The heartbeat is measured on a plurality of days until hatching, for example, every several days or every twenty-four hours or twelve hours. The egg weight is measured, for example, with a weight scale. The egg weight is measured on a plurality of days until hatching, for example, every several days or every twenty-four hours or twelve hours. The measured heartbeat and egg weight are recorded in temporary recording unit 22 in information processing apparatus 2 and transmitted over the network to egg management support system 1 as appropriate.

An inside temperature is measured with a thermometer attached to the inside of incubator 3. The inside temperature is measured, for example, every twenty-four hours or twelve hours. The measured inside temperature is recorded in temporary recording unit 22 in information processing apparatus 2 and transmitted over the network to egg management support system 1 as appropriate.

After eggs are transferred (egg transfer) from a setter to a hatcher on Day 18, the eggs are hatched around Day 21. As the eggs are hatched in the hatcher, a humidity increases and hence the hatch window is calculated based on information measured with a hygrometer attached to the inside of incubator 3. After hatching, chicks are classified in chick treatment apparatus 5. In chick treatment apparatus 5, chicks are counted and a hatching rate is calculated. In chick treatment apparatus 5, chick quality is converted into a numeric form with the use of images. The hatch window, the hatching rate, and the chick quality are recorded in temporary recording unit 22 of information processing apparatus 2 and transmitted over the network to egg management support system 1. The egg ID or the tray ID is used to associate such incubation performance with biological information.

In egg management support system 1, the biological information, the incubation performance, the attribute information, and the environmental information received from a plurality of hatcheries 4 by receiver 11 are stored for each egg ID. A user who can access egg management support system 1 can make use of various types of information stored in egg management support system 1.

Exemplary usage includes control of an inside environment in incubator 3 based on the environment control information generated in information generator 13 of egg management support system 1. The environment control information is calculated by information generator 13 based on information about change in biological information and change in environmental information for improving incubation performance that is stored in storage 12.

As described above, egg management support system 1 according to the present embodiment includes receiver 11 and storage 12. Receiver 11 receives from information processing apparatuses 2 at a plurality of hatcheries 4, biological information of eggs measured on a plurality of days before hatching and incubation performance obtained for chicks that have hatched out of the eggs. The biological information and the incubation performance are stored in storage 12 in association. Thus, information on a plurality of hatcheries 4 can effectively be made use of.

Egg management support system 1 further includes information generator 13 that generates environment control information for controlling an inside environment in incubator 3 based on the biological information and the incubation performance. Therefore, even a worker without the know-how can appropriately control incubator 3 to improve incubation performance.

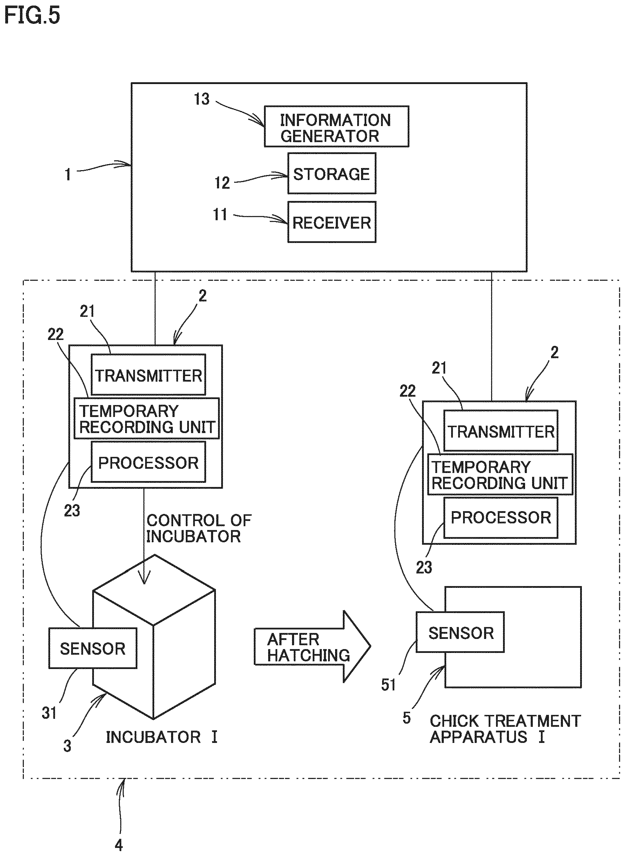

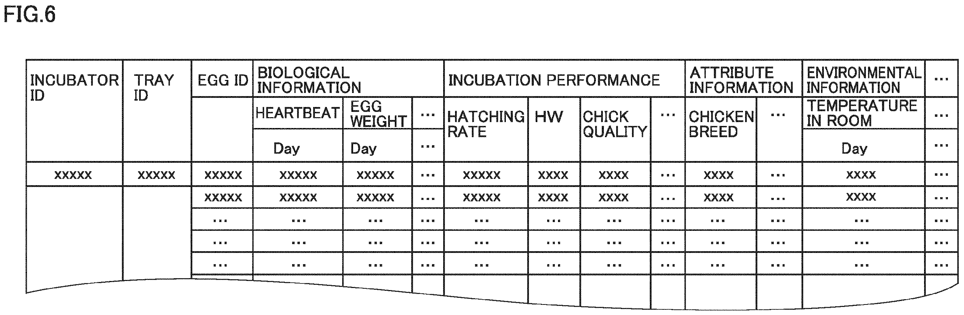

The present invention is not limited to the embodiment described above. A modification of the present embodiment will be described with reference to Figs. 5 and 6.

As shown in Fig. 5, egg management support system 1 may manage biological information and incubation performance of eggs at a single hatchery 4 (single farm). In other words, ecological information and incubation performance of eggs at single hatchery 4 are stored in association in this egg management support system 1.

The ecological information of eggs is measured for eggs within single incubator 3 provided at single hatchery 4. Alternatively, the ecological information of eggs is measured for eggs within a plurality of incubators 3 provided at single hatchery 4.

The incubation performance is obtained for chicks that have hatched out of eggs at single hatchery 4 and treated in single treatment apparatus 5. Alternatively, the incubation performance is obtained for chicks that have hatched out of eggs at single hatchery 4 and treated in a plurality of treatment apparatuses 5.

As shown in Fig. 6, egg management support system 1 may measure biological information of eggs for one specific day before hatching. The ecological information and the incubation performance of eggs at single hatchery 4 or a plurality of hatcheries 4 are stored in association in egg management support system 1. The ecological information of eggs is measured for each of eggs in the incubator on one specific day before hatching. The incubation performance is obtained for chicks that have hatched out of the eggs.

For one specific day, in Fig. 6, a day of measurement of a heartbeat, a day of measurement of an egg weight, and a day of measurement of an inside temperature are each denoted as "Day". The day of measurement may be the same for a plurality of measurement items or different for each of the plurality of measurement items. For example, the day of measurement of the heartbeat may be Day 18, the day of measurement of the egg weight may be Day 0, and the day of measurement of the inside temperature may be Day 10.

Similarly, even when a plurality of days of measurement are set, the number of times of measurement or timing of measurement of each measurement item may be the same or different.

Storage 12 is not limited to a storage provided on a cloud but any storage where biological information and incubation information can be stored in association may be applicable. Storage 12 may be a storage where various types of information are received via electronic mails and stored.

The biological information is not limited to biological information measured with eggs being carried on a tray in incubator 3 but may be measured with the tray having been taken out of incubator 3 or with eggs having been taken out of the tray. The biological information may be measured, for example, in transfer of eggs from a setter to a hatcher.

The embodiment disclosed herein is illustrative and limitation thereto is not intended. The present invention is defined by the terms of the claims rather than the scope described above, and is intended to include any modifications within the scope and meaning equivalent to the terms of the claims.

### INDUSTRIAL APPLICABILITY

The present invention can be made use of for an egg management support system.

### REFERENCE SIGNS LIST

1 egg management support system; 11 receiver; 12 storage; 13 information generator; 2 information processing apparatus; 4 hatchery

## Claims

1. An egg management support system comprising:
a receiver that receives from an information processing apparatus at a hatchery, biological information of an egg measured before hatching and incubation performance obtained for a chick that has hatched out of the egg; and
a storage where the biological information and the incubation performance are stored in association.

2. The egg management support system according to claim 1, further comprising an information generator that generates environment control information for controlling an inside environment of an incubator based on the biological information and the incubation performance.
